# EUROPEAN PATENT APPLICATION

(11) **EP 3 821 933 A1**
(43) Date of publication of application: **19.05.2021**
(21) Application number: 19220019.4
(22) Date of filing: 30.12.2019
(51) Int. Cl.: A61M 16/10, A61M 16/20, A61M 16/00

(54) **GAS VALVE SYSTEM WITH FLOW SENSOR**

(30) Priority: 18.11.2019 EP 19209878
(71) Applicant: Oxypoint NV, 2018 Antwerpen (BE)
(72) Inventor: HENDRICKX, Philip, 2600 Berchem (BE); VAN STEENKISTE, Cedric, 2547 Lint (BE); PINTENS, Niels, 2900 Schoten (BE); BORGONJON, Dirk, 2950 Kapellen (BE); VAN ROOST, Wouter, 1981 Hofstade (BE); HERMANS, Paul, 2660 Hoboken (BE)
(74) Representative: DenK iP bv

(57) **Abstract**

A gas valve system (100) adapted for controlling the flow of a medical gas for a user, the gas valve system (100) comprising
- a housing (260),
- one or more sensors (216, 218, 220),
- one or more electronic and/or mechanical components (240, 242) for controlling the gas flow, and
- a manifold (200) comprising one or more channels for guiding the medical gas between an input (212) and an output (222) of the gas valve system (100) and to which the one or more sensors(216, 218, 220) and one or more electronic and/or mechanical components (240, 242) can be coupled.

## Description

### Field of the invention

The invention relates to the medical field. More particularly, the present invention relates to systems and methods for administering medical gas to a patient.

### Background of the invention

The use of gases in medical applications is widespread. One of the gasses often administered to patients for medical reasons is oxygen. In view of the fact that administering oxygen is often essential for preventing damage to tissue, avoiding life-threatening situations or saving a patient from a life-threatening situation, hospitals distribute oxygen using a pipeline network up to the bed of nearly each patient. Furthermore, oxygen therapy is also widespread for homecare patients, making use of pressurised oxygen bottles and or oxygen concentrators. In both cases, a gas valve system is used for controlling the flow, i.e. between 1 I/min and 15 I/min or higher, and the type of delivery, i.e. continuous flow or on demand flow.

A plurality of gas valve systems is available on the market, both in full mechanical as well as in combined mechanical and electronic configurations.

Current gas valve systems typically comprise a number of components, mechanical and/or electronic components whereby the gas flow in the gas valve system is led from an entrance point to an exit point by tubes passing these different mechanical and/or electronic components. The assembly of such gas valve systems requires quite some effort.

### Summary of the invention

It is an object of embodiments of the present invention to provide good gas valve systems and methods of using them.

It is an advantage of embodiments of the present invention that systems are made that allow easy manufacturing.

The present invention relates to a gas valve system adapted for controlling the flow of a medical gas for a user, the gas valve system comprising a housing, one or more sensors, one or more electronic and/or mechanical components for controlling the gas flow, and a manifold comprising one or more channels for guiding the medical gas between an input and an output of the gas valve system and to which the one or more sensors and one or more electronic and/or mechanical components can be coupled.

It is an advantage of embodiments of the present invention that the internal gas flow in the gas valve system is guided through a manifold rather than to separate tubings, allowing a more easy assembling or the gas valves and more reliable system. It is an advantage of embodiment of the present invention that a rigid system is obtained.

The channels may have an average diameter of between 0.1mm and 10mm, for example between 1mm and 5mm.

One or more of the channels in the manifold may comprise one or more local channel diameter reductions, also referred to as orifice ring. It is an advantage of embodiments of the present invention that there is no need for a separate raster component which typically needs to be introduced in the gas tubing for allowing accurate operation of the gas valve system.

The local channel diameter reduction, also referred to as orifice ring, may be provided by local protrusions of the channel wall into the channel. It is an advantage of embodiments of the present invention that local channel diameter reductions can be easily introduced during the production process of the manifold.

The channel may comprise openings in the channel wall before and after the local channel diameter reduction, to which a flow sensor can be coupled for measuring the flow of the medical gas in a bypass channel. It is an advantage of embodiments according to the present invention that by providing local channel diameter reductions, the system can be used with a smaller sensor for measuring the flow and/or smaller electronic and/or mechanical components.

The openings in the channel wall may have an axial direction perpendicular to the overall longitudinal direction of the gas channel.

The openings in the channel wall may have an axial direction non-perpendicular to the overall longitudinal direction of the gas channel.

The manifold may be made of a single piece comprising the channels. The manifold may be made in any suitable manner, such as for example by moulding, by 3D printing, by processing a block of material, etc.

The manifold may be made of any suitable material, such as for example it may for example be made of a plastic or a metal.

The manifold may comprise alignment features for aligning one of the one or more sensors and/or one or more electronic or mechanical components with respect to the channels in the manifold. It is an advantage of embodiments of the present invention that easy positioning of the different components can be obtained during assembly of the gas valve system.

The present invention also relates to a manifold for use in a gas valve system, the manifold comprising one or more channels for guiding a medical gas between an input and an output of the gas valve system and to which the one or more sensors and one or more electronic and/or mechanical components can be coupled . The one or more channels in the manifold may comprise one or more local channel diameter reductions, also referred to as orifice ring.

The local channel diameter reduction may be provided by local protrusions of the channel wall into the channel.

The channel may comprise openings in the channel wall before and after the local channel diameter reduction, to which a flow sensor can be coupled for measuring the flow of the medical gas in a bypass channel.

The openings in the channel wall may have an axial direction perpendicular to the overall longitudinal direction of the gas channel or wherein the openings in the channel wall have an axial direction non-perpendicular to the overall longitudinal direction of the gas channel.

The present invention also relates to a flow sensor for measurement of a gas flow, the flow sensor comprising one or more channels for guiding the gas between an input and an output of the one or more channels, wherein the one or more channels comprise one or more local channel diameter reductions and wherein the channel comprises openings in the channel wall before and after the local channel diameter reduction, to which a flow sensor can be coupled for measuring the flow of the medical gas in a bypass channel.

Particular and preferred aspects of the invention are set out in the accompanying independent and dependent claims. Features from the dependent claims may be combined with features of the independent claims and with features of other dependent claims as appropriate and not merely as explicitly set out in the claims.

These and other aspects of the invention will be apparent from and elucidated with reference to the embodiment(s) described hereinafter.

### Brief description of the drawings

FIG. 1 illustrates an elevated top view of a system according to an embodiment of the present invention.
FIG. 2 to 7 illustrate different side views, top and bottom views and cross-sectional views of the system as indicated in FIG. 1;
FIG. 8 illustrates the manifold with additional components, as can be used in an embodiment according to the present invention.
FIG. 9 illustrates a manifold with connectors according to an embodiment of the present invention.
FIG 10 illustrates a gas valve system according to an embodiment of the present invention.
FIG. 11 illustrates a flow sensor according to an embodiment of an aspect of the present invention.
FIG. 12 illustrates another example of a gas valve system according to an embodiment of the present invention.
FIG. 13 illustrates the principle of a gas pressure reducing element, as used in embodiments of the present invention.
FIG. 14 and 15 illustrate a connector or connector add-on having a built in gas pressure reducing element, according to embodiments of the present invention.

The drawings are only schematic and are non-limiting. In the drawings, the size of some of the elements may be exaggerated and not drawn on scale for illustrative purposes.

Any reference signs in the claims shall not be construed as limiting the scope.

In the different drawings, the same reference signs refer to the same or analogous elements.

### Detailed description of illustrative embodiments

The present invention will be described with respect to particular embodiments and with reference to certain drawings but the invention is not limited thereto but only by the claims. The dimensions and the relative dimensions do not correspond to actual reductions to practice of the invention.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.
In a first aspect, the present invention relates to a gas valve system adapted for controlling the flow of a medical gas for a user. The gas valve system according to embodiments of the present invention may be a gas valve system for delivering continuous gas flow to a patient, for administering gas flow on demand, or for allowing both continuous gas administering or on demand gas administering. The gas valve system is not limited to a system for a particular type of gas. Depending on the properties of the gas to be delivered, the different settings of the components may be adapted. Furthermore, also the particularities of the design of the different components may be adapted. The gas valve system according to an aspect of the present invention comprises a housing. Such a housing may be made in any suitable material and may be of any suitable shape. In the housing, one ore more sensors such as for example an absolute pressure sensor, a flow sensor, a differential pressure sensor, etc. The gas valve system also comprises one or more electronic or mechanical components, such as for example valves like needle valves, for controlling the amount of gas flow that is to be administered. The gas valve system also may comprise an electronic or mechanical input means for allowing a user to provide the required input.
According to embodiments of the present invention, the gas valve system also comprises a manifold comprising one or more channels for guiding the medical gas between an input and an output of the gas valve system and to which the one or more sensors and one or more electronic and/or mechanical components can be coupled. The channels in the manifold may be fixed channels formed in the material of the manifold. The channels may have a diameter between 0.1mm and 10mm, e.g. between 0.5mm and 5mm. The cross-sectional shape of the channels may depend on the manufacturing technique of the manifold. The channels may have a square cross section, a rectangular cross section, a circular cross-section etc.
The manifold may be made of a single piece comprising the channels, but also may comprise of a plurality of pieces. In some embodiments, two major parts of the manifold may be present, whereby the two parts fit together and can be mounted to form a single piece. Alternatively, the manifold also could be made of a single piece. The manifold may be made in a plurality of manners, such as for example using moulding, using casting, using processing of a block of material, using 3D printing, and alike. The manifold may be made of any suitable material such as for example made of plastic, of metal, etc.
The manifold also may comprise alignment features for aligning one of the one or more sensors and/or one or more electronic or mechanical components with respect to the channels in the manifold.
The gas valve system also may comprise an output means for displaying information regarding the gas delivery. The displaying means may be a display for graphically or numerically displaying information. The displaying means may be a visual indication, such as for example a LED ring. In some embodiments, the gas valve system may comprise one or more channels in the manifold having one or more local channel diameter reductions, e.g. for obtaining flow reductions or measuring a flow. Such a local channel diameter reduction may overcome the need for using a honeycomb material in the channel, as typically is required in conventional systems for creating a differential pressure measurement system. The local channel diameter reduction may be provided by local protrusions of the channel wall into the channel. The channel may comprise openings in the channel wall before and after the local channel diameter reduction, to which a flow sensor can be coupled for measuring the flow of the medical gas in a bypass channel. The opening in the channel wall may have an axial direction perpendicular to the overall longitudinal direction of the gas channel or they may have an axial direction non-perpendicular to the overall longitudinal direction of the gas channel. The angle with respect to the overall longitudinal direction of the gas channel may be such that the amount of disturbance of the gas flow in the main channel is limited.

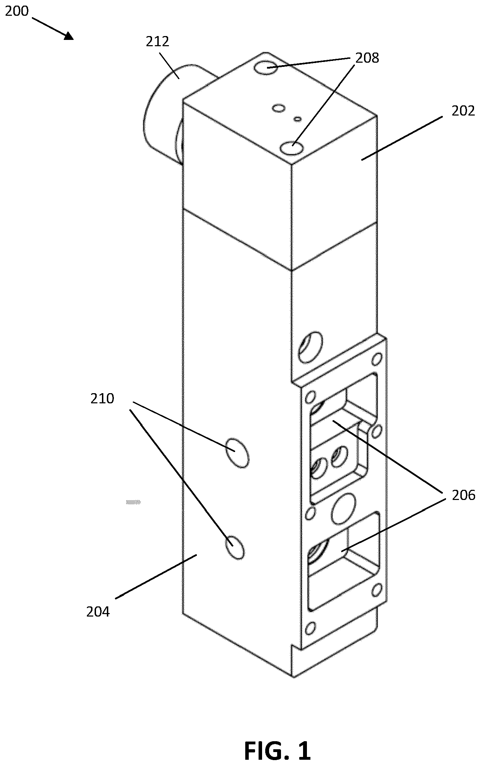

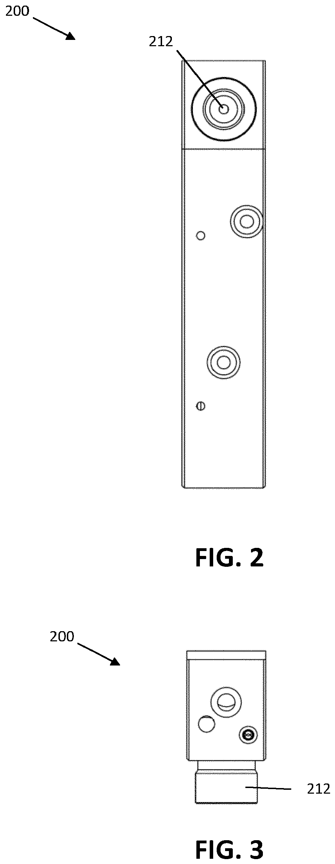

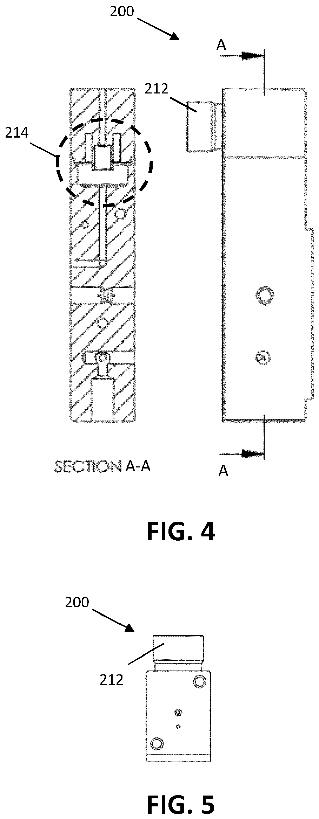

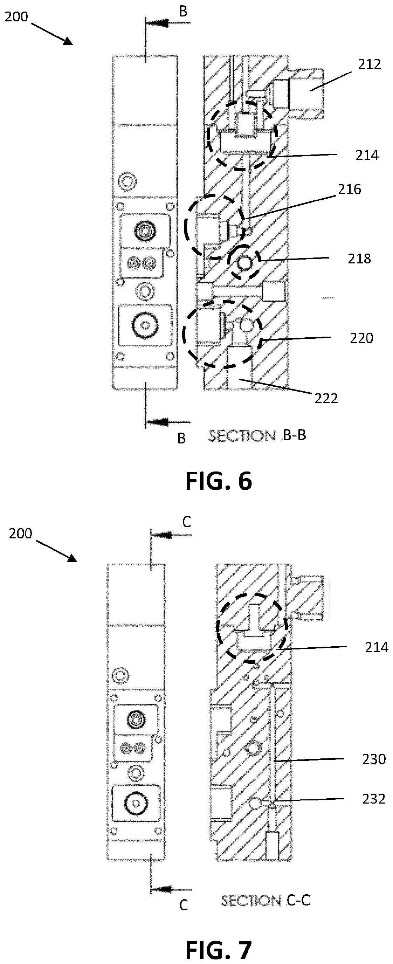

By way of illustration, embodiments of the present invention not being limited thereto, a gas valve system with manifold is described with reference to drawings FIG. 1 to FIG. 10. FIG. 1 shows an elevated view of the manifold 200. It can be seen that in the present example, the manifold 200 is built from two parts 202, 204, which allows for an easy assembly of the pressure reduction component that is present in the manifold and that will be discussed later. In FIG. 1, furthermore the positions 206 where the different sensors will be positioned can be seen. It is an advantage of embodiments of the present invention that the predefined upstanding edges and shaped features allow for an accurate positioning of the different sensors. Furthermore, mounting holes 208, 210 are shown that can be used for mounting different components to each other and/or for positioning the manifold with respect to the housing. FIG. 1 also illustrates a manifold input part 212 that is adapted for introducing the gas into the manifold. The manifold input part 212, may comprise in one example a threaded part for connecting a standard input connector to it. Such a standard input connector may be a DIN input connector, although embodiments are not limited thereto and FIG. 2 illustrates a back view of the manifold 200. The manifold input part 212 can be easily seen. FIG. 3 illustrates a bottom view of the manifold 200. Again the manifold input part 212 can be easily seen. Id. FIG. 4 illustrates a side view of the manifold 200 as well as a cross-sectional view along line A-A. In the cross-sectional view, part of the pressure reduction component 214 is shown, where the pressure of the incoming gas is reduce, so as to obtain a suitable pressure for use in gas delivery. The pressure reduction may for example be towards a predetermined pressure, such as for example to a pressure in a range between 1 bar and 2 bar, such as for example 1.5 bar or for example 2 bar. FIG. 5 illustrates a top view of the manifold. wherein again the manifold input part 212 can be seen. FIG. 6 illustrates a front view of the manifold with a cross-section line B-B for which the cross-sectional view along line B-B is also shown. FIG. 7 illustrates the same front view of the manifold but with cross-section line C-C for which the cross-sectional view along line C-C is also shown. Based on these drawings, the typical flow of the gas can be described.
In FIG. 6, the manifold input part 212 can be seen along which the gas enters the manifold. As described above, this component typically may comprise a threaded portion. From the manifold input part 212, the gas is led via one or more channels towards a pressure reduction component 214 for reducing the pressure so as to obtain a pressure that is suitable for use in gas delivery. From the pressure reduction component 214, the gas is again led via one or more channels towards an absolute pressure sensor 216. This absolute pressure sensor 216 may allow for evaluating the absolute pressure for the gas. The gas is then further led through channels towards a flow sensor 218 for checking the flow that is provided and controlled by a piezo-electrical valve. The gas is then further directed to a differential pressure sensor 220. The gas is thereafter led to a manifold output part 222.
The cross-sectional view C-C of FIG. 7 allows to further illustrate the channel used as a safety channel 230 so that in case of non-functioning of one of the components at least a safety gas flow can be obtained. The flow thereof will be controlled by a needle valve 232.

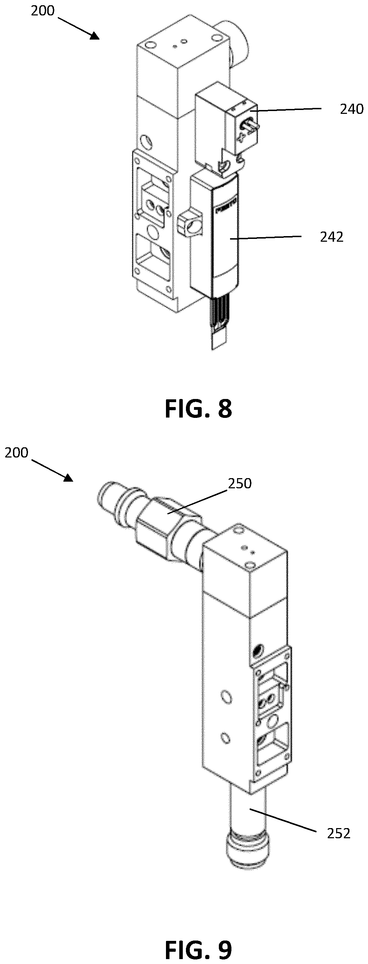

In FIG. 8, the pressure valves of which one 242 is used in the conventional gas stream for controlling the gas flow, which in the present example is a piezo-electric valve, and of which one 240 is used in the safety gas stream for controlling the gas flow, which in the present example is a latching valve, are shown, mounted on the manifold. The piezo-electric valve is provided with an electrical connector.
FIG. 9 illustrates an additional connector 250 which is mounted to manifold input part 212 and an additional connector 252 which is mounted to the manifold output part 222. The type of connectors used may depend on the type of gas connection requirements that are valid in the country of use of the gas valve. These connectors may allow for obtaining a country specific, conventional connection.

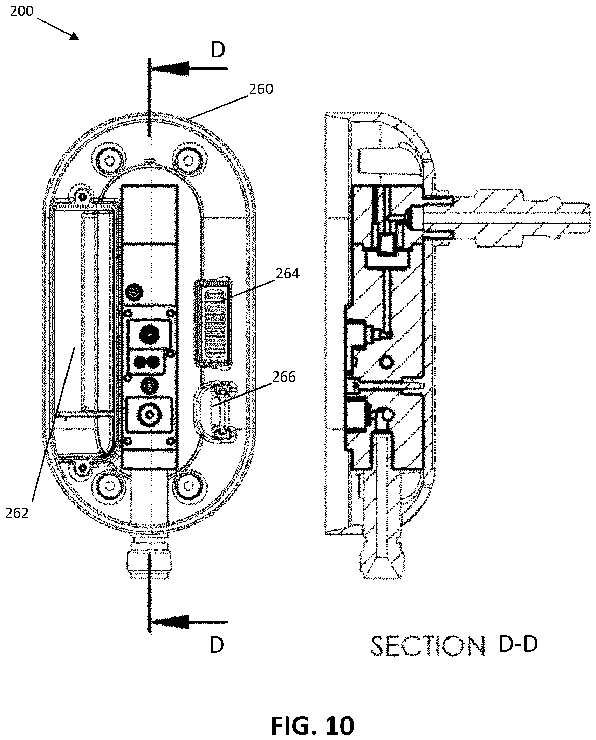

FIG. 10 illustrates how the manifold 200 is positioned in the housing 260 of the gas valve system 100. The manifold is positioned aside a space 262 where typically a battery is positioned. Furthermore, a speaker 264 and an usb connection 266 also is visible.

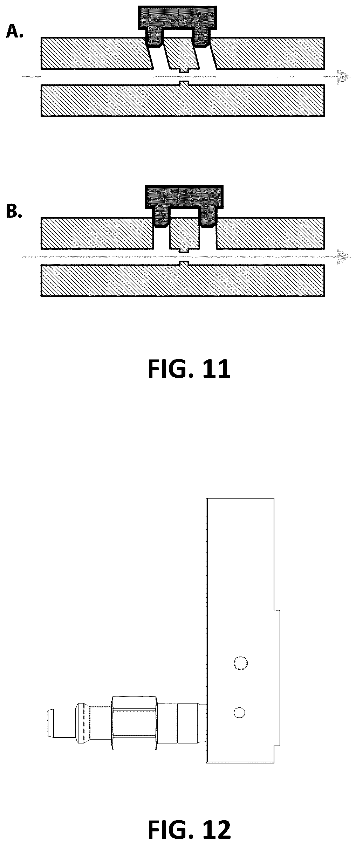

Further, by way of illustration, embodiments of the present invention not being limited thereto, an example of a specific flow sensor that can be used is now described. It is to be noted that the flow sensor can also be used in other gas valve systems and is therefore not limited to gas valve systems having a manifold according to embodiments according to the present invention.
In one aspect, the present invention thus also relates to a flow sensor based on a channel wherein one or more local reductions one or more local channel diameter reductions, e.g. for obtaining flow reductions or measuring a flow. Such a local channel diameter reduction may overcome the need for using a honeycomb material in the channel, as typically is required in conventional systems. The local channel diameter reduction may be provided by local protrusions of the channel wall into the channel. The channel may comprise openings in the channel wall before and after the local channel diameter reduction, to which a flow sensor can be coupled for measuring the flow of the medical gas in a bypass channel. The opening in the channel wall may have an axial direction perpendicular to the overall longitudinal direction of the gas channel or they may have an axial direction non-perpendicular to the overall longitudinal direction of the gas channel. The angle with respect to the overall longitudinal direction of the gas channel may be such that the amount of disturbance of the gas flow in the main channel is limited. By way of illustration, embodiments of the present invention not being limited thereto, two examples A and B of such a configuration is shown in FIG. 11.
In one embodiment, a flow sensor for measurement of a gas flow is described. The flow sensor comprises one or more channels for guiding the gas between an input and an output of the one or more channels. The one or more channels comprise one or more local channel diameter reductions and the channel comprises openings in the channel wall before and after the local channel diameter reduction, to which a flow sensor can be coupled for measuring the flow of the medical gas in a bypass channel. In a particular example, the orifice ring or channel diameter reduction reduces from a diameter of 4.7mm to 3.5mm and enlarges again to 4.7mm.

In one aspect, the present invention also relates to a manifold as described in the first aspect, Similar features as described for the first aspect may be present, leading to the same advantages.

By way of illustration, embodiments of the present invention not being limited thereto, a further example of a gas valve system is illustrated in FIG. 12. The gas valve system is similar to the gas valve systems as described above, but the manifold input part is positioned at a relative different position compared to the examples already illustrated. In the present example, the manifold input part is positioned more at a bottom side of the gas valve system. This illustrates that the exact position where the manifold input part is positioned relative to the housing is not limited. The inner components of the manifold may be rearranged with respect to the housing, or may be as illustrated in the examples above whereby an additional connection tube or channel between the manifold input part and for example a pressure reduction component, or if the pressure reduction component is positioned outside the gas valve system, a first sensor of the gas valve system.
In some embodiments, the manifold input part may be shiftable with respect to the housing, so that the relative position can be adjusted depending on the local environment and/or application.

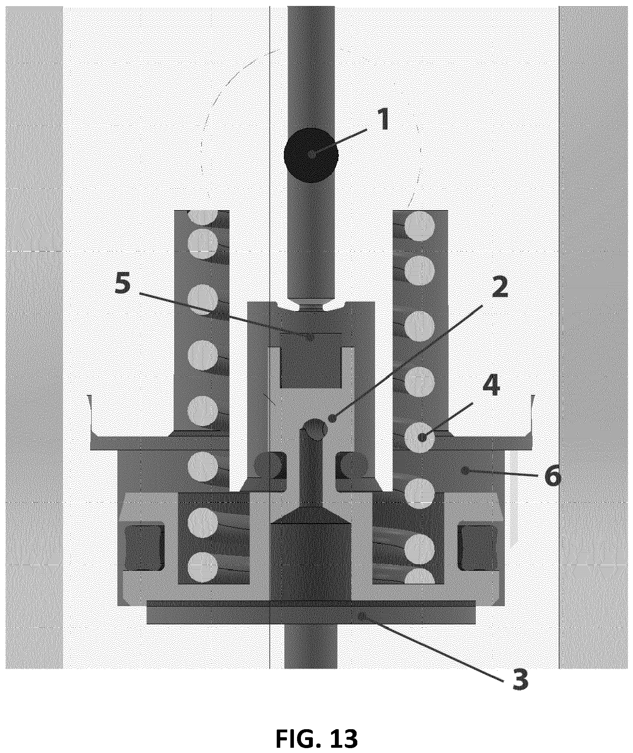

Also by way of illustration, embodiments not being limited thereto, an example of a pressure reduction component is described in more detail, with reference to FIG. 13. The gas reduction component has the function of bringing the gas pressure to a pressure that can be used for gas therapy. The working principle of the pressure reduction component may be as followed. The gas inlet 1 allows the gas to enter at its typical input pressure, which may be around 5 bar or around 6 bar depending on local conditions. The gas flows to he actuator via channel 2 being a connection in the actuator to the space 3 in the actuator. In this room the pressure builds up till a predetermined pressure sufficiently high for use in gas valve therapy, such as for example 2 bar. When the predetermined pressure is reached, a calibrated spring 4 is pressed, such that the gas flow is limited by a closing element 5, such as for example a rubber stopping element. The calibrated spring allows for the relative movement of the closing element 5 with respect to the gas inlet 1. This process occurs fast and repetitively. In this way, the pressure in the channels can be controlled and the pressure that is delivered is determined by the calibrated spring, i.e. by the power required for pressing the calibrated spring. The space 6 is in contact with the environment. The appropriate gas input pressure and output pressure can be obtained based on selection of the proper spring and proper dimensions of the system.

It is to be noted that one aspect of the present invention, which may be considered in combination with the manifold based gas pressure valve but also with any other type of gas valve system, relates to a gas valve system wherein a gas pressure reduction component is provided allowing reducing the gas pressure from between10 bar and 3 bar, e.g. from between 6 bar and 3 bar, e.g. from about 5 bar or 6 bar, to a predetermined gas pressure for use in gas therapy or other gas delivery applications. The system may be adapted for using a gas reduction component as the one described above. It is an advantage that an additional gas reduction component is provided, aside from the gas pressure reduction members that are already present when using pressurized gas bottles or gas distribution networks, since this allows for a more accurate predetermined level at which gas delivery such as gas therapy, can be performed. It furthermore is to be noted that also further gas pressure reducing elements may be present, used in series.

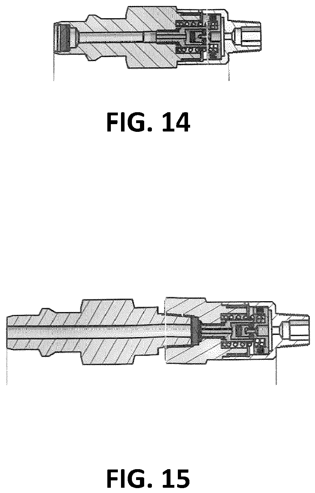

Whereas the gas pressure reduction element may be part of the gas valve system, it also may in one aspect be introduced in a connector element. In one aspect, the present invention thus also relates to a connector element for coupling a gas valve system to a pressurized gas bottle or a gas distribution network system, the connector element comprising a pressure reducing element. The latter is illustrated in FIG. 14 and 15, whereby the connector can be a single connector or an additional connector that can be mounted, e.g. clicked or screwed, on an existing connector. It is an advantage of embodiments of the present invention that a pressure reducing component can be embedded in a connector. The latter allows for a compact and user friendly manner for introducing a gas pressure reducing component. It is an advantage of embodiments of the present invention that in this way an easy double step pressure reduction can be obtained, one being provided by the conventional gas reduction element typically mounted on a gas bottle or a distribution network, and one provided by the gas pressure reducing element in the connector element. Furthermore, it can be easily seen that different connectors with gas pressure reducing elements can be coupled in series.

## Claims

1. A gas valve system (100) adapted for controlling the flow of a medical gas for a user, the gas valve system (100) comprising
- a housing (260),
- one or more sensors (216, 218, 220),
- one or more electronic and/or mechanical components (240, 242) for controlling the gas flow, and
- a manifold (200) comprising one or more channels for guiding the medical gas between an input (212) and an output (222) of the gas valve system (100) and to which the one or more sensors(216, 218, 220) and one or more electronic and/or mechanical components (240, 242) can be coupled.

2. A gas valve system (100) according to claim 1, wherein one or more of the channels in the manifold (200) comprise one or more local channel diameter reductions.

3. A gas valve system (100) according to any of the previous claims, wherein the local channel diameter reduction is provided by local protrusions of the channel wall into the channel.

4. A gas valve system (100) according to the previous claim, wherein the channel comprises openings in the channel wall before and after the local channel diameter reduction, to which a flow sensor (216) can be coupled for measuring the flow of the medical gas in a bypass channel.

5. A gas valve system (100) according to the previous claim, wherein the openings in the channel wall have an axial direction perpendicular to the overall longitudinal direction of the gas channel.

6. A gas valve system (100) according to the previous claim, wherein the openings in the channel wall have an axial direction non-perpendicular to the overall longitudinal direction of the gas channel.

7. A gas valve system (100) according to any of the previous claims, wherein the manifold (200) is made of a single piece or a combination of pieces comprising the channels.

8. A gas valve system (100) according to any of the previous claims, wherein the manifold (200) is made of any of a plastic or a metal.

9. A gas valve system (100) according to any of the previous claims, wherein the manifold (200) comprises alignment features for aligning one of the one or more sensors and/or one or more electronic or mechanical components (240, 242) with respect to the channels in the manifold (200).

10. A manifold (200) for use in a gas valve system (100), the manifold (200) comprising one or more channels for guiding a medical gas between an input (212) and an output (222) of the gas valve system (100) and to which the one or more sensors(216, 218, 220) and one or more electronic and/or mechanical components (240, 242) can be coupled .

11. A manifold (200) according to claim 10, wherein the one or more channels in the manifold (200) comprise one or more local channel diameter reductions.

12. A manifold (200) according to claim 11, wherein the local channel diameter reduction is provided by local protrusions of the channel wall into the channel.

13. A manifold (200) according to the previous claim, wherein the channel comprises openings in the channel wall before and after the local channel diameter reduction, to which a flow sensor (216) can be coupled for measuring the flow of the medical gas in a bypass channel.

14. A manifold (200) according to the previous claim, wherein the openings in the channel wall have an axial direction perpendicular to the overall longitudinal direction of the gas channel or wherein the openings in the channel wall have an axial direction non-perpendicular to the overall longitudinal direction of the gas channel.

15. A flow sensor for measurement of a gas flow, the flow sensor comprising one or more channels for guiding the gas between an input (212) and an output (222) of the one or more channels, wherein the one or more channels comprise one or more local channel diameter reductions and wherein the channel comprises openings in the channel wall before and after the local channel diameter reduction, to which a flow sensor (216) can be coupled for measuring the flow of the medical gas in a bypass channel.
